Europäisches Patentamt

(19) European Patent Office

Office européen des brevets

(11) Numéro de publication: **0 093 668 B1**

## FASCICULE DE BREVET EUROPEEN

(12)

(45) Date de publication du fascicule du brevet:
23.10.85

(51) Int. Cl.⁴: **B 01 J 13/02,** A 61 K 9/50

(21) Numéro de dépôt: **83400862.5**

(22) Date de dépôt: **29.04.83**

(54) Microcapsules à paroi obtenue par réticulation des protéines du lait, et leur procédé de préparation.

(30) Priorité: **04.05.82 FR 8207735**

(43) Date de publication de la demande:
**09.11.83 Bulletin 83/45**

(45) Mention de la délivrance du brevet:
**23.10.85 Bulletin 85/43**

(84) Etats contractants désignés:
**AT BE CH DE GB IT LI LU NL SE**

(56) Documents cités:
**FR - A - 2 251 366**
**FR - A - 2 444 497**
**US - A - 3 137 631**

(73) Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Levy, Marie-Christine, 18 ter, rue Houzeau-Muivon, F-51100 Reims (FR)**
Inventeur: **Guerin, Danièle, 14 rue des Pressoirs Bourgogne, F-51220 Hermonville (FR)**

(74) Mandataire: **Ahner, Francis et al, CABINET REGIMBEAU 26, avenue Kléber, F-75116 Paris (FR)**

BUNDESDRUCKEREI BERLIN

## Description

La présente invention concerne des microcapsules à paroi obtenue par réticulation des protéines du lait ainsi que leur procédé de préparation. L'invention se rapporte à des microcapsules renfermant en particulier, mais non exclusivement, une substance pharmaceutiquement active. Les microcapsules selon l'invention peuvent en effet également renfermer d'autres substances telles que des substances alimentaires et en particulier des huiles essentielles.

On rappellera tout d'abord brièvement que les microcapsules sont des organites artificiels qui présentent un très grand intérêt pour la mis en forme galénique de divers médicaments. L'inclusion d'un principe actif dans une sphérule microscopique permet en effet d'assurer sa protection transitoire vis-à-vis d'agents dénaturants, tels que les enzymes digestives. Il est nécessaire que la paroi de la sphérule présente toutes les garanties d'innocuité, et de biodégradabilité liées à l'emploi en thérapeutique humaine.

A ce jour, il n'existe pas de procédé de microencapsulation qui s'applique indifféremment à des principes actifs hydrophiles et à des principes actifs lipophiles.

On notera également que, si des formes pharmaceutiques gastrorésistantes solides (comprimés, gélules) ont été assez largement développées à ce jour, des formes liquides gastrorésistantes (suspensions), qui seraient nécessaires pour la thérapeutique infantile, n'ont pas été développées.

L'objet de la présente invention permet notamment de résoudre les problèmes évoqués ci-dessus.

Les microcapsules conformes à la présente invention sont caractérisées en ce qu'elles comportent une paroi externe obtenue par réticulation interfaciale des protéines du lait à l'aide d'un agent de réticulation en particulier constitué par un réactif bifonctionnel acylant ou par un dialdéhyde tel que le glutaraldéhyde.

Conformément à la présente invention de telles microcapsules peuvent être obtenues à partir du lait par mise en œuvre des trois étapes successives suivantes:

i)  le lait, préalablement additonné à la substance pharmaceutiquement active, est émulsifié par dispersion au sein d'un solvant organique non miscible, par addition d'un agent émulsifiant et par agitation;

ii)  à l'émulsion ainsi obtenue, maintenue sous agitation, on ajoute un agent de réticulation en solution dans ledit solvant organique non miscible et l'on maintient l'agitation jusqu'à obtention d'une réticulation interfaciale, et

iii)  on isole les microcapsules par dilution du mélange réactionnel au moyen d'un sulvant ou d'un mélange de solvants approprié, suivie d'une succession d'étapes de décantation et/ou de centrifugation et d'étapes de lavage.

L'utilisation du lait de vache pour la fabrication de microcapsules présente un certain nombre d'avantages étroitement liés au fait qu'il s'agit d'une matière première abondante, naturelle, non toxique et biodégradable. Le lait de vache, qui est une émulsion lipidique au sein d'une solution aqueuse de protéines, apporte d'autres avantages décisifs dans cette utilisation particulière:

—  les qualités mécaniques des parois des microcapsules conformes à la présente invention sont considérablement améliorées,

—  les microcapsules préparées à partir du lait résistent à l'action des enzymes gastriques, et sont rapidement lysées par les enzymes du milieu intestinal. Elles se prêtent particulièrement bien à la protection de médicaments sous des formes gastrorésistantes entérosolubles, liquides telles que des suspensions de microcapsules, ou solides telles que des gélules contenant les microcapsules objet de l'invention,

—  l'utilisation du lait offre toute garantie d'innocuité de la matière première quant à son utilisation en thérapeutique humaine.

D'autres caractéristiques et avantages de la présente invention apparaîtront à la lecture de la description détaillée faite ci-après notamment en référence à quelques exemples particuliers donnés à titre de simple illustration.

La présente invention s'applique à la microencapsulation de substances pharmaceutiquement actives de n'importe quelle nature, parmi lesquelles on mentionnera à titre d'exemples les substances hydrosolubles, telles que des sels alcalins d'acides carboxyliques, par exemple l'acide salicylique, les sels d'un acide minéral ou organique et d'une base organique, par exemple le chlorhydrate d'amfépramone, ainsi que les substances huileuses et les substances liposolubles, telles que des huiles végétales, par exemple l'huile d'olive, des huiles animales, par exemple les huiles de foie de morue et de flétan, ou encore d'autres médicaments, par exemple le clofibrate.

Conformément à la présente invention, les microcapsules sont obtenues à partir du lait auquel on applique le procédé de microencapsulation par émulsion-réticulation, développé par M. C. LEVY et coll. RAMBOURG (P): »Sur la microencapsulation de l'invertase et de l'hémoglobine: de la polymérisation interfaciale au procédé d'émulsion-réticulation«. Thèse Doctorat, 3ème cycle, Université Paris-Sud, U.E.R. Chimie Thérapeutique, 1980 LEVY (M. C.), RAMBOURG (P) et LEVY (J), 2ème Congrès Int. Technol. Pharm. Paris 1980, III, 15—24 Microencapsulation III).

Le procédé de microencapsulation selon l'invention se subdivise en trois étapes successives, à savoir:

i)   une première étape d'émulsification du lait;
ii)  une deuxième étape de réticulation interfaciale, et
iii) une troisième étape d'isolement des microcapsules.

Au cours de la première étape d'émulsification du lait, on disperse le lait en fins globules au sein d'un solvant organique non miscible, grâce à l'adjonction d'un agent émulsifiant et en réalisant une agitation. La substance qui sera encapsulée est ajoutée préalablement au lait. Le lait utilisé est du lait de vache, éventuellement homogénéisé, entier ou partiellement écrémé. On emploie sans inconvénient du lait reconstitué à partir de lait concentré ou de poudre de lait, entier ou partiellement écrémé.

Le solvant organique utilisé au cours de cette première étape l'émulsification est avantageusement choisi de telle sorte qu'il ne soit pas miscible à l'eau, que sa densité soit sensiblement comprise entre 0,7 et 1,1, et que l'agent de réticulation utilisé dans la deuxième phase s'y dissolve. On emploie avec profit par exemple un mélange binaire constitué par un hydrocarbure chloré à un ou deux atomes de carbone tel que le chloroforme, le chlorure de méthylène ou le trichloréthylène et par un hydrocarbure non chloré comportant de 5 à 7 atomes de carbone, tel que l'hexane ou le cyclohexane.

L'agent émulsifiant utilisé au cours de cette première étape est un agent tensioactif tel qu'un ester de sorbitane et d'acide gras ou tout autre tensioactif choisi par l'homme de l'art en vue de préparer une émulsion de type eau dans huile. Le trioléate de sorbitane (Span 85®) est utilisé avec profit.

Les volumes et quantités relatifs de lait de solvant organique et d'agent émulsifiant permettant d'obtenir l'émulsion susceptible d'être ultérieurement réticulée peuvent être adaptés avec une assez grande latitude et s'éloigner notablement des proportions indiquées dans les exemples décrits ci-après.

Une telle émulsion peut aisément être obtenue par agitation, par exemple au moyen d'une ancre de verre entraînée mécaniquement à l'intérieur d'un tube à centrifugation. Une vitesse de rotation comprise entre environ 1000 et environ 2000 tours par minute, poursuivie pendant au moins environ trois minutes suffit alors pour mener à bien l'émulsification.

Selon une variante du procédé de l'invention, l'étape d'émulsification du lait peut être précédée par la réalisation d'une très fine émulsion primaire d'une huile, végétale ou animale dans le lait. A cet effet, on utilise alors un agitateur mécanique permettant une vitesse de rotation supérieure, par exemple avantageusement comprise entre environ 10 000 et environ 20 000 tours par minute, et l'huile est émulsionnée dans le lait en présence d'un agent émulsifiant choisi par l'homme de l'art en vue d'obtenir une émulsion de type huile dans eau, par exemple le polysorbate (Tween 20®), ou la lécithine de soja. Dans ce cas, on utilise avec profit du lait reconstitué préparé à partir de lait en poudre écrémé.

Cette variante s'applique également à une huile contenant en solution une substance liposoluble et à un principe actif huileux.

La très fine émulsion primaire d'huile dans le lait est ensuite utilisée de la même manière que le lait dans la première étape d'émulsification, décrite ci-avant.

Dans la deuxième étape de réticulation interfaciale, la paroi des microcapsules est formée par réticulation en particulier au moyen d'un réactif bifonctionnel acylant, tel qu'un halogénure de diacide carboxylique, un diisocyanate, où les deux groupements fonctionnels sont tous deux portés par un cycle aromatique, ou séparés par une chaîne aliphatique de 2 à 10 atomes de carbone, ou portés, l'un par un cycle aromatique, l'autre par une chaîne aliphatique juxtanucléaire, par exemple, le dichlorure de succinyle, le dichlorure de sébacoyle, le dichlorure de téréphtaloyle, le diisocyanate de toluène, le diisocyanate d'hexaméthylène, ou encore par un dialdéhyde tel que le glutaraldéhyde.

L'agent de réticulation est ajouté, en solution de préférence dans le solvant organique déjà utilisé dans la première étape, à l'émulsion de lait maintenue sous agitation.

Cette opération est réalisée à une température comprise entre le point de congélation et le point d'ébullition des liquides utilisés, le plus souvent à température ordinaire.

Il convient de noter que, conformément à la présente invention, il n'est pas indispensable de stabiliser le pH du mélange réactionnel au cours de cette deuxième opération, grâce au pouvoir tampon du lait utilisé comme matière première.

Une concentration de l'agent de réticulation dans le mélange solvant variant de la saturation à une concentration 0,1 M est compatible avec l'obtention de microcapsules. Des conditions favorables à la préparation des microcapsules sont généralement obtenues lorsque la solution de l'agent de réticulation est ajoutée à l'émulsion de lait sous un volume sensiblement égal au volume de solvant ayant servi à préparer ladite émulsion.

L'agitation est poursuivie jusqu'à ce qu'un examen au microscope optique montre la formation de microcapsules individualisées, ce qui nécessite en moyenne une durée de l'ordre d'environ 30 minutes.

La troisième étape d'isolement des microcapsules est réalisée en diluant le mélange réactionnel au moyen d'un solvant ou d'un mélange de solvants, puis en soumettant la suspension de microcapsules à une succession de décantations et de lavages.

La décantation peut être avantageusement remplacée par une centrifugation, par exemple 350 × g, 30 secondes.

Le lavage des microcapsules est effectué dans un solvant choisi pour ne pas entraîner les substances microencapsulées:

— Dans le cas de substances hydrosolubles, on utilise par exemple l'éther éthylique ou l'alcool éthylique.
— Dans le cas des huiles et des substances liposolubles, on utilise par exemple l'eau distillée éventuellement additionnée de polysorbate (Tween 20®).

Les microcapsules sèches sont obtenues par évaporation de l'éther ou de l'alcool lorsque l'un de ces solvants a été utilisé dans la phase ultime du lavage, ou encore par lyophilisation d'une suspension de microcapsules dans l'eau, soumise préalablement à une congélation.

Les microcapsules préparées à partir du lait selon les caractéristiques de l'invention, se présentent en microscopie optique, sous forme de sphérules bien individualisées, généralement de couleur jaune, dont le diamètre peut varier entre 25 et 300 microns.

Des sphérules de taille plus grande peuvent être obtenues lorsque les opérations d'émulsification et de réticulation sont effectuées avec une vitesse d'agitation inférieure à 1000 tours par minute.

Une membrane épaisse d'environ 1 à environ 2 microns est visible. Lorsque les microcapsules sont préparées à partir de lait entier, ou lorsqu'une émulsion primaire d'une huile dans le lait écrémé a été réalisée selon la variante indiquée ci-avant, elles contiennent de fins globules lipidiques d'un diamètre d'environ 1 à environ 3 microns.

En microscopie électronique à balayage, elles montrent une paroi lisse et continue.

Les microcapsules lyophilisées ont des parois collapsées et reprennent rapidement une forme sphérique par réhydratation.

Placées dans un milieu gastrique artificiel, les microcapsules préparées à partir de lait et d'un chlorure de diacide tel que le chlorure de téréphtaloyle ne présentent aucune altération microscopiquement visibles après 3 heures à 37°C. Après 8 heures de contact, 70% environ des microcapsules sont encore intactes. En revanche, la paroi de toutes les microcapsules est lysée en moins de 5 minutes dans un milieu intestinal artificiel. Ces propriétés s'appliquent à la préparation de médicaments sous une forme gastrorésistante entérosoluble.

Les exemples suivants illustrent le procédé objet de la présente invention, sans en limiter toutefois les applications.

## Exemple 1

### Microcapsules préparées à partir de lait entier homogénéisé

Toutes les opérations sont conduites à température ordinaire.

### 1. Emulsification

Dans un tube à centrifuger de 55 ml (diamètre intérieur 3,1 cm) sont placés 15 ml d'un mélange chloroforme-cyclohexane (1 : 4 v/v) — ci-après appelé mélange solvant — additionné de 0,3 ml de trioléate de sorbitane (Span 85®). On ajoute 3 ml de lait entier homogénéisé et on agite mécaniquement (1300 tours par minute) au moyen d'une ancre de verre.

### 2. Réticulation

Après 3 minutes, et sans interrompre l'agitation, on ajoute 15 ml d'une solution saturée de chlorure de téréphtaloyle dans le solvant. L'agitation est poursuivie 30 minutes puis arrêtée.

### 3. Isolement des microcapsules

Le contenu du tube à centrifuger est transféré dans un bécher contenant 30 ml de mélange solvant. Les microcapsules formées sont dispersées par agitation manuelle au moyen d'un agitateur de verre pendant 1 à 2 minutes. Cette suspension est soumise à une centrifugation (350 × g, 30 secondes). Le culot de centrifugation est remis en suspension dans 20 ml d'alcool à 95°, on agite manuellement (agitateur de verre) 1 à 2 minutes et on centrifuge à nouveau dans les mêmes conditions. Il es procédé à trois opérations identiques de lavage à l'alcool. A l'issue de celles-ci, le culot de centrifugation est mis en suspension dans 100 ml d'eau distillée.

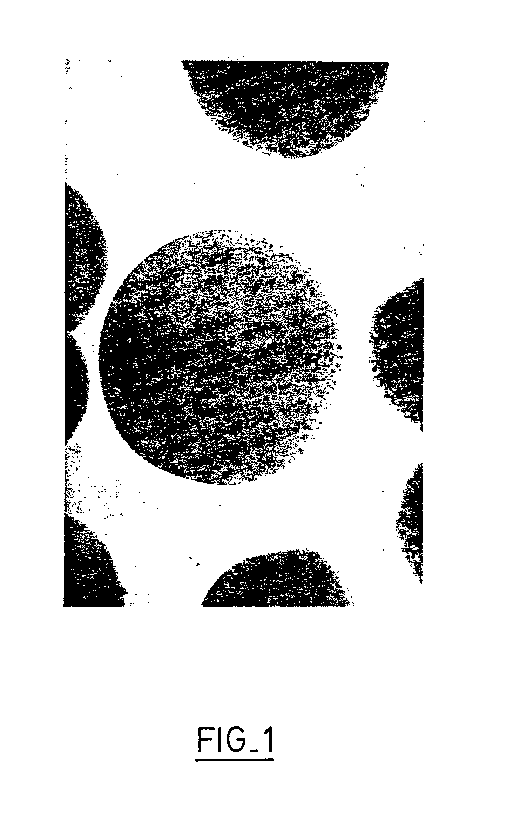

Les microcapsules obtenues se présentent en microscopie optique sous forme de sphères régulières et bien individualisées d'un diamètre de 25 à 300 microns, colorées en jaune. Elles contiennent les lipides du lait sous forme de fins globules d'un diamètre de 1 à 2 microns. La figure 1 représente un cliché de microscopie optique (grossissement 300 fois) des microcapsules obtenues conformément à l'exemple 1.

### 4. Lyophilisation

La suspension aqueuse précédente est congelée à −10° et lyophilisée. On obtient 0,75 g d'une poudre de microcapsules.

### 5. Essai de gastrorésistance

50 mg de poudre de microcapsules lyophilisées sont mis en suspension dans 1 ml d'eau et ajoutés à 15 ml de liquide gastrique artificiel à pH 1,2 (U.S.P. XIX), au bain-marie à 37°.

L'examen microscopique optique montre que la paroi des microcapsules n'est pas altérée après 3 heures. Après 8 heures, 70% des microcapsules ont encore conservé leur intégrité.

## 6. Essai de solubilité entérique

Un essai pratiqué comme au cinquièmement, en remplaçant le liquide gastrique artificiel par le liquide intestinal artificiel pH 7,5 (U.S.P. XIX) montre que la paroi de tous les lots de capsules étudiés est totalement lysée entre 2 et 4 minutes.

## Exemple 2

### Microcapsules préparées à partir de lait reconstitué

Le lait est reconstitué par addition d'eau dans les proportions prescrites par le fabricant. Une variante consiste à utiliser des quantités d'eau proportionnellement moins importantes, ce qui donne des microcapsules de plus grande densité. C'est ainsi que la quantité d'eau ajoutée peut être diminuée jusqu'à 4 ml pour 1 g de poudre de lait entier, et jusqu'à 3 ml pour 1 g de poudre de lait écrémé.

Le protocole décrit à l'exemple 1 est ensuite appliqué.

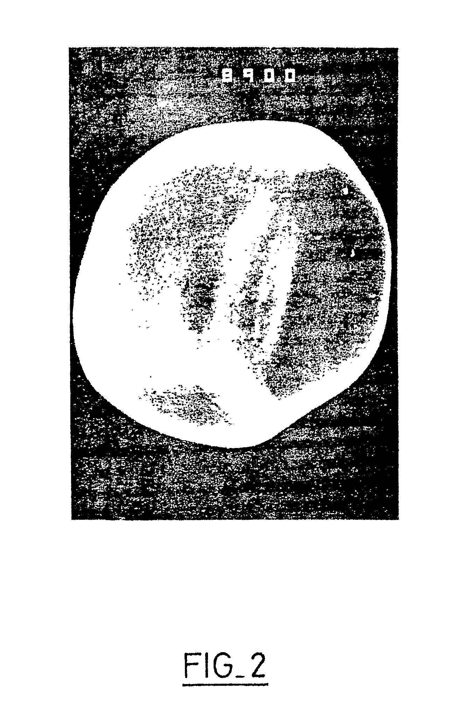

La figure 2 représente un cliché de microscopie électronique à balayage (grossissement 1000 fois) des microcapsules préparées à partir de lait reconstitué conformément à l'exemple 2.

## Exemple 3

### Microcapsules contenant du salicylate de sodium

#### 1. Emulsification

Le protocole décrit à l'exemple 1 est reproduit, en remplaçant ici les 3 ml de lait entier par 3 ml de lait reconstitué (1 g de lait écrémé + 4 ml d'eau) contenant en solution 100 mg de salicylate de sodium.

#### 2. Réticulation

Le protocole décrit à l'exemple 1 est reproduit.

#### 3. Isolement des microcapsules

Le contenu du tube à centrifuger est transféré dans un bécher contenant 30 ml de mélange solvant. Les microcapsules sont dispersées par agitation manuelle au moyen d'un agitateur de verre pendant 1 à 2 minutes. Cette suspension est soumise à une centrifugation (350 × g, 30 secondes). Le culot de centrifugation est remis en suspension dans 20 ml d'éther, agité manuellement (agitateur de verre) 1 à 2 minutes et soumis à une décantation. Le surnageant est éliminé. Les microcapsules sont lavées une nouvelle fois à l'éther puis séchées à l'air libre.

## Exemple 4

### Microcapsules contenant du chlorhydrate d'amfépramone

Le protocole décrit à l'exemple 3 est reproduit en remplaçant, lors de l'émulsification, le salicylate de sodium par 100 mg de chlorhydrate d'amfépramone.

## Exemple 5

### Microcapsules contenant de l'huile d'olive

#### 1. Emulsification primaire

Dans le récipient de verre d'un homogénéisateur VIRTIS® 23, sont placés 40 g de lait écrémé reconstitué (10 g de poudre + 30 ml d'eau) additionnés de 0,6 ml de polysorbate (Tween 20®) et de 3 ml d'huile d'olive.

On agite 6 minutes à 13 000 tours par minute à l'aide d'une double lame métallique. On obtient ainsi une émulsion très fine d'huile dans le lait. Au microscope optique, les globules d'huile ont une taille de 1 à 2 microns.

#### 2. Microencapsulation

3 ml de cette émulsion fine sont soumis à la microencapsulation selon le protocole décrit dans l'exemple précédent.

## Exemple 6

### Microcapsules contenant de l'huile d'olive

Le protocole décrit à l'exemple 5 est reproduit en remplaçant le polysorbate par de la lécithine de soja (0,6 g). Les microcapsules obtenues sont lavées au moyen d'une solution à 5% (v/v) de polysorbate (Tween 20®) dans l'eau distillée. Après 3 lavages successifs, le culot de centrifugation est lavé à l'eau pure et la suspension de microcapsules dans l'eau est soumise à la lyophilisation.

## Exemple 7

### Microcapsules contenant du clofibrate

Le protocole décrit à l'exemple 5 est reproduit en remplaçant l'huile d'olive par 3 ml de clofibrate.

Les microcapsules obtenues sont lyophilisées. On obtient ainsi une poudre de microcapsules de couleur jaune pâle.

Au microscope optique, on distingue les globules de clofibrate (1 à 2 microns) à l'intérieur des microcapsules.

Bien entendu, la présente invention n'est nullement limitée aux exemples particuliers de mise en œuvre précédemment décrits, mais il est parfaitement possible, sans pour autant sortir du cadre de la présente invention, d'en imaginer un certain nombre de variantes d'exécution.

**Revendications**

1. Microcapsules renfermant une substance pharmaceutiquement active, caractérisées en ce que la paroi externe des microcapsules est obtenue par réticulation interfaciale des protéines du lait à l'aide d'un agent de réticulation en particulier constitué par un réactif bifonctionnel acylant ou par un dialdéhyde tel que le glutaraldéhyde.

2. Microcapsules selon la revendication 1, caractérisées en ce que leur diamètre moyen est sensiblement compris entre 25 et 300 microns.

3. Microcapsules selon l'une des revendications 1 et 2, caractérisées en ce qu'elles présentent une paroi continue d'épaisseur sensiblement comprise entre 1 et 2 microns.

4. Microcapsules selon l'une des revendications 1 à 3, caractérisées en ce qu'elles contiennent une substance pharmaceutiquement active constituée par des substances hydrosolubles, des substances huileuses ou des substances liposolubles.

5. Procédé de préparation de microcapsules renfermant une substance pharmaceutiquement active, caractérisé en ce que l'on prépare les microcapsules à partir du lait par mise en œuvre des trois étapes successives suivantes:

i) le lait, préalablement additionné à la substance pharmaceutiquement active, est émulsifié par dispersion au sein d'un solvant organique non miscible, par addition d'un agent émulsifiant et par agitation;

ii) à l'émulsion ainsi obtenue, maintenue sous agitation, on ajoute un agent de réticulation en solution dans ledit solvant organique non miscible et l'on maintient l'agitation jusqu'à obtention d'une réticulation interfaciale, et

iii) on isole les microcapsules par dilution du mélange réactionnel au moyen d'un solvant ou d'un mélange de solvants approprié, suivie d'une succession d'étapes de décantation et/ou de centrifugation et d'étapes de lavage.

6. Procédé selon la revendication 5, caractérisé en ce que l'on utilise du lait homogénéisé entier ou partiellement écrémé ou encore du lait reconstitué par addition d'eau à partir de lait en poudre entier ou partiellement écrémé.

7. Procédé selon l'une des revendications 5 et 6, caractérisé en ce que le solvant organique utilisé au cours de l'étape d'émulsification est un solvant non miscible à l'eau, de densité sensiblement comprise entre 0,7 et 1,1, et permettant la dissolution de l'agent de réticulation utilisé au cours de la seconde étape de réticulation interfaciale.

8. Procédé selon l'une des revendications 5 à 7, caractérisé en ce que l'agent émulsifiant utilisé au cours de l'étape d'émulsification est un agent tensioactif permettant la préparation d'une émulsion du type eau dans huile.

9. Procédé selon l'une des revendications 5 à 8, caractérisé en ce que ledit solvant organique non miscible est constitué par un mélange binaire d'un hydrocarbure chloré en $C_1$ ou $C_2$ et d'un hydrocarbure non chloré en $C_5$ à $C_7$.

10. Procédé selon l'une des revendications 5 à 9, caractérisé en ce que la première étape d'émulsification du lait est précédée par la réalisation d'une très fine émulsion primaire d'une huile végétale ou animale dans le lait.

11. Procédé selon la revendication 10, caractérisé en ce que ladite émulsion primaire est réalisée en présence d'un agent tensioactif permettant l'obtention d'une émulsion de type huile dans eau.

12. Procédé selon l'une des revendications 5 à 11, caractérisé en ce que l'agent de réticulation est un réactif bifonctionnel acylant, tel qu'un halogénure de diacide carboxylique, un diisocyanate, où les deux groupements fonctionnels sont tous deux portés par un cycle aromatique ou séparés par une chaîne aliphatique contenant de 2 à 10 atomes de carbone, ou portés, l'un par un cycle aromatique et l'autre par une chaîne aliphatique juxtanucléaire, ou encore un dialdéhyde.

13. Procédé selon l'une des revendications 5 à 11, caractérisé en ce que l'agent de réticulation est choisi parmi le dichlorure de succinyle, le dichlorure de sébacoyle, le dichlorure de téréphtaloyle, le diisocyanate de toluène, le diisocyanate d'hexaméthylène et le glutaraldéhyde.

14. Procédé selon l'une des revendications 5 à 13, caractérisé en ce que l'agent de réticulation est ajouté au mélange réactionnel en solution dans le même solvant organique que celui utilisé au cours de la première étape d'émulsification.

15. Procédé selon l'une des revendications 12 à 14, caractérisé en ce que la concentration de l'agent de réticulation dans ledit solvant organique varie de la saturation à une concentration 0,1 M.

16. Procédé selon la revendication 15, caractérisé en ce que la solution d'agent de réticulation est ajoutée à l'émulsion de lait à raison d'un volume sensiblement égal au volume de solvant utilisé pour la préparation de ladite émulsion.

17. Procédé selon l'une des revendications 5 à 16, caractérisé en ce que les microcapsules sont récupérées à l'état sec, soit par évaporation du ou des solvants de lavage, soit par lyophilisation de la suspension des microcapsules dans l'eau, soumise préalablement à une congélation.

**Patentansprüche**

1. Mikrokapseln, die eine pharmazeutisch aktive Substanz einschließen, dadurch gekennzeichnet, daß die Außenwand der Mikrokapseln durch Grenzflächenvernetzung der Milchproteine mit Hilfe eines Vernetzungsmittels hergestellt wird, insbesondere ein bifunktionelles acylierendes Reagenz oder durch ein Dialdehyd, wie zum Beispiel Glutaraldehyd.

2. Mikrokapseln nach Anspruch 1, dadurch gekennzeichnet, daß ihr durchschnittlicher Durchmesser im wesentlichen zwischen 25 und 300 Mikron liegt.

3. Mikrokapseln nach einem der Ansprüche 1 und 2, dadurch gekennzeichnet, daß sie eine durchgehende Wand aufweisen, deren Stärke im wesentlichen zwischen 1 und 2 μ liegt.

4. Mikrokapseln nach einem der Ansprüche 1 bis 3, dadurch gekennzeichnet, daß sie eine pharmazeutisch aktive Substanz enthalten, die von wasserlöslichen Substanzen, öligen Substanzen oder fettlöslichen Substanzen gebildet wird.

5. Verfahren zur Herstellung von Mikrokapseln, die eine pharmazeutisch aktive Substanz einschließen, dadurch gekennzeichnet, daß die Mikrokapseln unter Durchführung von drei aufeinanderfolgenden Schritten aus Milch hergestellt werden:

i)   die Milch, die zuerst der pharmazeutisch aktiven Substanz beigemengt wird, wird durch Dispersion in einem nicht mischbaren organischen Lösungsmittel durch Zusatz eines Emulgiermittels und durch heftige Bewegung emulgiert;

ii)   der so erhaltenen, in Bewegung gehaltenen Emulsion wird ein Vernetzungsmittel in Lösung im genannten organischen nicht mischbaren Lösungsmittel zugesetzt, und die heftige Bewegung wird aufrechterhalten bis eine Grenzflächenvernetzung eintritt, und

iii)   die Mikrokapseln werden durch Verdünnen des Reaktionsgemisches mit Hilfe eines Lösungsmittels oder einer geeigneten Lösungsmittelmischung getrennt, gefolgt von einer Reihe von Arbeitsschritten, wie Dekantieren und/oder Zentrifugieren und Waschen.

6. Verfahren nach Anspruch 5, dadurch gekennzeichnet, daß homogenisierte Vollmilch oder teilweise entrahmte Milch oder auch durch Zusatz von Wasser rekonstituierte Milch aus Vollmilchpulver oder teilweise entrahmtem Milchpulver verwendet wird.

7. Verfahren nach einem der Ansprüche 5 und 6, dadurch gekennzeichnet, daß das beim Emulgieren verwendete organische Lösungsmittel ein mit Wasser nicht mischbares Lösungsmittel ist, dessen Dichte im wesentlichen zwischen 0,7 und 1,1 liegt, und in dem das im zweiten Grenzflächenvernetzungsschritt verwendete Vernetzungsmittel löslich ist.

8. Verfahren nach einem der Ansprüche 5 bis 7, dadurch gekennzeichnet, daß das beim Emulgieren verwendete Emulgierungsmittel ein Tensid ist, das die Herstellung einer Wasser-in-Öl-Emulsion erlaubt.

9. Verfahren nach einem der Ansprüche 5 bis 8, dadurch gekennzeichnet, daß das genannte organische nicht mischbare Lösungsmittel von einer binären Mischung eines $C_1$- oder $C_2$-chlorierten Kohlenwasserstoffs und eines $C_5$- bis $C_7$-nicht-chlorierten Kohlenwasserstoffs gebildet wird.

10. Verfahren nach einem der Ansprüche 5 bis 9, dadurch gekennzeichnet, daß dem ersten Emulgieren der Milch die Herstellung einer sehr feinen Primäremulsion eines Pflanzen- oder Tieröls in der Milch vorausgeht.

11. Verfahren nach Anspruch 10, dadurch gekennzeichnet, daß die genannte Primäremulsion in Gegenwart eines Tensids erfolgt, das die Herstellung einer Öl-in-Wasser-Emulsion erlaubt.

12. Verfahren nach einem der Ansprüche 5 bis 11, dadurch gekennzeichnet, daß das Vernetzungsmittel ein bifunktionelles acylierendes Reagenz ist, wie zum Beispiel ein Dicarbonsäurehalogenid, ein Diisocyanat, wobei sich die beiden funktionellen Gruppen an einem aromatischen Ring befinden oder durch eine aliphatische Kette enthaltend 2 bis 10 Kohlenstoffatome getrennt sind, oder die eine sich an einem aromatischen Ring und die andere sich an einer juxtanuklearen aliphatischen Kette befindet, oder auch ein Dialdehyd ist.

13. Verfahren nach einem der Ansprüche 5 bis 11, dadurch gekennzeichnet, daß das Vernetzungsmittel aus Succinyldichlorid, Sebacoyldichlorid, Terephthaloyldichlorid, Toluoldiisocyanat, Hexamethylendiisocyanat und Glutaraldehyd ausgewählt wird.

14. Verfahren nach einem der Ansprüche 5 bis 13, dadurch gekennzeichnet, daß das Vernetzungsmittel dem Reaktionsgemisch in Lösung im gleichen organischen Lösungsmittel zugesetzt wird, das im ersten Emulgiervorgang verwendet wurde.

15. Verfahren nach einem der Ansprüche 12 bis 14, dadurch gekennzeichnet, daß die Konzentration des Vernetzungsmittels im genannten organischen Lösungsmittel zwischen Sättigung und einer Konzentration von 0,1 M variiert.

16. Verfahren nach Anspruch 15, dadurch gekennzeichnet, daß die Lösung des Vernetzungsmittels der Milchemulsion in einer Volumenmenge zugesetzt wird, die im wesentlichen gleich ist wie das Volumen des für die Herstellung der genannten Emulsion verwendeten Lösungsmittels.

17. Verfahren nach einem der Ansprüche 5 bis 16, dadurch gekennzeichnet, daß die Mikrokapseln in trockenem Zustand gewonnen werden, entweder durch Verdampfen der Waschlösung(en) oder durch Lyophilisation der Suspension der Mikrokapseln in Wasser, wobei diese vorher eingefroren wird.

**Claims**

1. Microcapsules enclosing a pharmaceutically active substance, characterised in that the external wall of the microcapsules is obtained by interfacial reticulation of proteins of milk with the aid of a reticulation agent in particular constituted by a bifunctional acylation reagent or by a dialdehyde such as glutaraldehyde.

2. Microcapsules according to claim 1, characterised in that their mean diameter is substantially between 25 and 300 microns.

3. Microcapsules according to claim 1 or claim 2, characterised in that they have a continuous wall of thickness substantially between 1 and 2 microns.

4. Microcapsules according to any one of claims 1 to 3, characterised in that they contain a pharmaceutically active substance taken from water soluble substances, oily substances or liposoluble substances.

5. A method of preparation of microcapsules enclosing a pharmaceutically active substance, characterised in that the microcapsules are prepared from milk by employing the three successive following stages:

i)    the milk, previously added to the pharmaceutically active substance, is emulsified by dispersion within it of a non-miscible organic solvent, by the addition of an emulsifying agent and by shaking;

ii)   to the emulsion thus obtained, still being shaken, a reticulation agent is added in solution into the said non-miscible organic solvent and the shaking is maintained until interfacial reticulation is obtained, and

iii)  the microcapsules are isolated by dilution of the reactive mixture by means of a solvent or an appropriate mixture of solvents, followed by a succession of separation stages and/or centrifuging and washing stages.

6. A method according to claim 5, characterised in that full cream or partly skimmed milk or again milk reconstituted by the addition of water to powdered milk from full cream or partly skimmed milk is used.

7. A method according to claim 5 or claim 6, characterised in that the organic solvent used in the emulsification stage is a solvent not miscible with water, of a density substantially between 0.7 and 1.1, and permitting the dissolution of the reticulation agent used in the second stage of interfacial reticulation.

8. A method according to any one of claims 5 to 7, characterised in that the emulsifying agent used in the emulsification stage is a surface active agent permitting the preparation of a water in oil type of emulsion.

9. A method according to any one of claims 5 to 8, characterised in that the said non-miscible organic solvent is constituted by a binary mixture of a $C_1$ or $C_2$ chlorinated hydrocarbon and a $C_5$ to $C_7$ non-chlorinated hydrocarbon.

10. A method according to any one of claims 5 to 9, characterised in that the first stage of emulsification of the milk is preceded by the production of a very fine primary emulsion of a vegetable or animal oil in the milk.

11. A method according to claim 10, characterised in that the said primary emulsion is produced in the presence of a surface active agent permitting the obtaining of an oil in water type of emulsion.

12. A method according to any one of claims 5 to 11, characterised in that the reticulation agent is a bifunctional acylation reagent, such as a halide of carboxylic diacid, a diisocyanate, where the two functional groups are both carried by an aromatic ring or separated by an aliphatic chain containing 2 to 10 carbon atoms, or carried, one by an aromatic ring and the other by a juxtanuclear aliphatic chain, or again a dialdehyde.

13. A method according to any one of claims 5 to 11, characterised in that the reticulation agent is chosen from succinyl dichloride, sebacoyl dichloride terephtaloyl dichloride, toluene diisocyanate, hexamethylene diisocyanate and glutaraldehyde.

14. A method according to any one of claims 5 to 13, characterised in that the reticulation agent is added to the reactive mixture in solution in the same organic solvent which itself was used in the first emulsification stage.

15. A method according to any one of claims 12 to 14, characterised in that the concentration of the reticulation agent in the said organic solvent varies between saturation and a 0.1 M concentration.

16. A method according to claim 15, characterised in that the reticulation agent is added to the emulsion of milk in a volume ratio substantially equal to the volume of solvent used for the preparation of the said emulsion.

17. A method according to any one of claims 5 to 16, characterised in that the microcapsules are recovered to a dry state, either by evaporation of the or each washing solvent, or by lyophilisation of the suspension of the microcapsules in water, previously congealed.

FIG_1

FIG_2

11